(19) Europäisches Patentamt
European Patent Office
Office européen des brevets

(11) Veröffentlichungsnummer: **0 124 041 B2**

(12) # NEUE EUROPÄISCHE PATENTSCHRIFT

(45) Veröffentlichungstag der neuen Patentschrift: **06.11.91 Patentblatt 91/45**

(21) Anmeldenummer: **84104475.3**

(22) Anmeldetag: **19.04.84**

(51) Int. Cl.$^5$: **C07C 45/68,** C07C 45/65, C07C 69/757, C07C 67/347, C07C 49/713, C07C 49/747, C07C 49/403, C07D 213/46

(54) **Verfahren zur Herstellung von Cyclohexandionderivaten.**

Teilanmeldung 88120492.9 eingereicht am 19/04/84.

(30) Priorität: **23.04.83 DE 3314816**

(43) Veröffentlichungstag der Anmeldung: **07.11.84 Patentblatt 84/45**

(45) Bekanntmachung des Hinweises auf die Patenterteilung: **21.11.85 Patentblatt 85/47**

(45) Bekanntmachung des Hinweises auf die Entscheidung über den Einspruch: **06.11.91 Patentblatt 91/45**

(84) Benannte Vertragsstaaten: **AT BE CH DE FR GB IT LI NL**

(56) Entgegenhaltungen:
**EP-B- 0 061 669**
**EP-B- 0 066 195**
**JP-A-79 630 52**
**US-A- 2 592 890**
**US-A- 3 950 420**
**US-A- 4 281 204**
**CHEMICAL ABSTRACTS, Band 88, 1978, Seite 466, Nr. 74127k, Columbus, Ohio, USA**
**"Guide for the perplexed organic experimentalist". H J E Loewwnthal. Heyden & son, 1978 p.82**
**Akhrem et al. Synthesis 1978 pp 925-927**
**Translation of pp 18-23 of paper by M Sawaki & Takahashi. Nikkakyo Geppo 1980**
**Vogels text book of Practical Organic Chemistry 4th edition. Longman pp 856-857**
**Organic Syntheses collective vol. 4. John Wiley & Sons, pp. 285-287**
**Riegel & Lilienfield. Journal of American Chem. Soc. 1945 vol. 67 pp. 1273-1275**
**Reactions of organic compounds. Longmans Green & Co. London pp 94-95, 128-129**
**Chem. abstracts 91 (1979). Ref. 1992911w**
**Heterogeneous reactions, analysis, examples, reactor design. Vol. 2. Doraiswamy & Sharma. John Wiley & Sons**
**Chem. engineers handbook 5th edition, Perry & Chilton. McGraw Hill.**

(56) Entgegenhaltungen:
**Proceedings of the Fifth International Congress of Pesticide Chemistry, 1982, Volume 1, pages 151 to 158, published by Pergamon, Oxford, 1983**

(73) Patentinhaber: **BASF Aktiengesellschaft**
**Carl-Bosch-Strasse 38**
**W-6700 Ludwigshafen (DE)**

(72) Erfinder: **Reissenweber, Gernot, Dr.**
**Drosselstrasse 15**
**W-6737 Boehl-Iggelheim (DE)**
Erfinder: **Richarz, Winfried, Dr.**
**Koenigsberger Strasse 5**
**W-6081 Stockstadt (DE)**

**EP 0 124 041 B2**

## Beschreibung

Die Erfindung betrifft ein Verfahren zur Herstellung von Cyclohexandionderivaten (I)

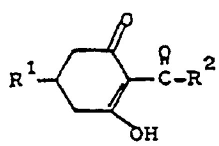

(I)

die als unmittelbare Vorprodukte zur Herstellung von Mitteln zur Beeinflussung des Pflanzenwachstums geeignet sind. Solche Mittel sind beispielsweise in der DE-OS 2 822 304 und in den US-PSen 3 950 420 und 4 011 256 beschrieben. Dabei ist angegeben, daß die Mittel aus Vorprodukten des Typs (I) erhälthich sind.

Nach Tetrahedron Lett. 29, 249 (1975) und Synthesis 1978. 925 können Verbindungen (I) aus entsprechend substituierten Cyclohexandionen durch C- bzw. O-Acylierung erhalten werden, wobei die O-Acylierung einen anschließenden Isomerisierungsschritt erfordert.

Soweit man den angegebenen Stellen entnehmen kann, ist die jeweils erzielbare Einheitlichkeit der Umsetzung und demnach die Ausbeute der einzelnen Schritte nicht besonders hoch. Die Eigenart der jeweils beteiligten Reaktionen erfordert außerdem jeweils die Reingewinnung der Zwischenprodukte, was erfahrungsgemäß die Gesamtausbeute weiter verringert.

Es besteht daher Bedarf an einem einfachen, in möglichst einfachen Reaktionsgefäßen ausführbaren Verfahren zur Gewinnung von Cyclohexandionderivaten (I)

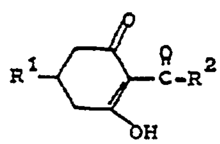

(I)

wobei $R^1$ einen der in Anspruch 1 angegebenen Reste bedeutet und $R^2$ einen Alkylrest mit bis zu 6 C-Atomen bedeutet.

Es wurde gefunden, daß man Verbindungen (I) ausgehend von einfachen, wohlfeilen Verbindungen, nämlich α,β-ungesättigten Ketonen (II)

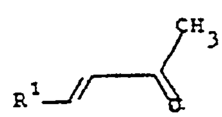

(II)

und Malonsäuredialkylestern in einem einzigen Reaktionsgefäß und einem gleichbleibenden Lösungs-mittel erhalten kann.

Erfindungsgemäß setzt man

1. ein entsprechendes α,β-ungesättigtes Keton der Formel II in Gegenwart von Alkalialkoholat

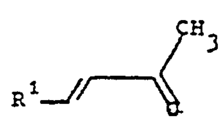

(II)

mit einem Malonsäuredialkylester in ein einem alkylierten oder halogenierten aromatischen Kolelenwasserstoff, aus dem das gebieldte salz ausfällt und, aus dem der freiwendende Alkohol des Malonesters abdestilliert werden kann, zu einem Alkoxycarbonylcyclohexenolon (III), bzw. dessen Salz

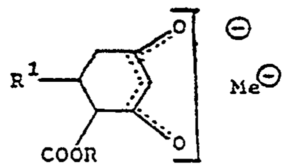

(III)

UM,

2. destilliert den Alkohol ggf. als Azeotrop mit einem Teil des Lösungsmittels ab,
3. versetzt das Salz des Cyclohexenolons III mit einem Carbonsäurehalogenid (IV)

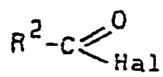

(IV)

wobei $R^2$ vorzugsweise Methyl, Ethyl oder Propyl, allgemein einen Alkylrest mit z.B. bis zu 6 C-Atomen bedeutet, wobei ein Gemisch von Cyclohexenoncarbonsäureestern (Va) bzw. (Vb) gebildet wird

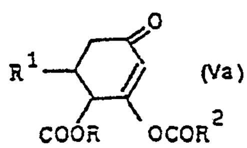

(Va)

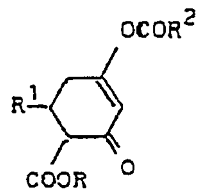

(Vb)

4. lagert das Gemisch der Ester (Va) und (Vb) mittels eines tertiären Amins als Umlagerungskatalysators zum 2-Acylcyclohexandion (VI)

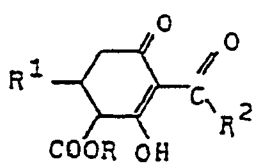

(VI)

UM,

5. verseift und decarboxyliert.

Das Verfahren ist anwendbar auf die Herstellung von Cyclohexandionderivaten der Formel I mit den oben angegebenen Substituenten $R^1$ und $R^2$. Oer Substituent R in dem vorstehenden Reaktionsschema entspricht dem Alkylrest des im Malonester eingebauten Alkohols und bedeutet aus wirtschaftlichen Gründen vorzugsweise Methyl oder Ethyl.

Als Lösungsmittel verwendet man einen alkylierten oder halogenierten aromatischen Kohlenwasserstoff wie Toluol, Ethylbenzol, ein Xylol, Isopropylbenzol, Chlorbenzol. In diesem Lösungsmittel fällt das Salz als fester Stoff, d.h. als Suspension aus. Dies ist für die Umsetzung typisch.

Toluol wird als Lösungsmittel bevorzugt, wenn Dimethylmalonat als Reaktionsteilnehmer verwendet wird, da Methanol mit Toluol ein Azeotrop bildet.

Das Verhältnis von ungesättigtem Keton und Malonester ist bevorzugt stöchiometrisch ; ein geringfügiger Überschuß des einen oder anderen ist nicht besonders störend; größerer Überschuß soll aber vermieden werden, weil es zu einem zusätzlichen Trennungsschritt führen, bzw. zusätzlichen Destillationsaufwand bedeuten würde.

Die Umsetzung verläuft schon bei Raumtemperatur und vervollständigt sich in dem Maße, wie der freigesetzte Alkohol durch allmähliches Erwärmen abdestilliert wird. Sie kann auch bei höherer Temperatur, z. B. bis zu 100°C vorgenommen werden.

Die Entfernung des Methanols bzw. Alkohols muß so vollständig wie möglich geschehen, da das zuzusetzende Acylhalogenid sonst bevorzugt mit dem Alkohol reagiert.

Die im wesentlichen alkoholfreie Suspension des gebildeten Salzes versetzt man nun mit dem Säurehalogenid und hält auf 40 bis 200, bevorzugt 80 bis 160°C, wobei das Salz in Lösung geht und das entsprechende Alkalihalogenid ausfällt. Auch das Säurehalogenid wird in etwa stöchiometrischer Menge, zweckmäßig nich im Überschuß verwendet.

Man setzt anschließend eine katalytische Menge eines Umlagerungskatalysators zu. Geeignete Katalysatoren sind tertiäre Amine und besonders Pyridinbasen wie 4-Dimethylaminopyridin, 4-Piperidinopyridin, 4-Morpholinopyridin oder N-alkylierte Imidazole bzw. Benzimidazole. Die Umlagerung verläuft im gleichen Temperaturbereich wie die vorhergehende, manchmal schon bei Raumtemperatur mit ausreichender Geschwindigkeit.

Die Vollständigkeit der Umlagerung kann z. B. durch Dünnschichtchromatographie geprüft werden.

Man setzt nunmehr wäßrige Alkalilauge zu und gewinnt das zu verseifende Produkt als Lösung in wäßriger Phase. Es versteht sich, daß man das Lösungsmittel auch vorher abziehen und den Rückstand in wäßriger Lauge aufnehmen kann. Die Verseifung benötigt i. a. eine geringere Temperatur als die vorausgehenden Umsetzungen, z. B. 20 bis 100, bevorzugt 40 bis 80 °C. Die Menge an Alkalilauge muß naturgemäß wenigstens 2 Moläquivalente, bezogen auf das zu Beginn eingesetzte Keton betragen, da sowohl die Alkoxycarbonylgruppe wie der bewegliche Wasserstoff des Diketons je 1 Moläquivalent Base binden. Ein gewisser Überschuß an Lauge beschleunigt die Verseifung, jedoch sollte ein zu großer Überschuß vermieden werden, da noch decarboxyliert werden muß.

Die Decarboxylierung verläuft in mineralsaurer Lösung oder in Gegenwart einer stärkeren Carbonsäure wie Ameisen- oder Essigsäure zwischen Raumtemperatur und etwa 100°C.

Das gesuchte Cyclohexandionderivat fällt i. a. aus der wäßrigen, sauren Lösung aus und kann durch Filtration gesammelt, evtl. auch extrahiert und ggf. durch Umkristallisieren gereinigt werden.

Die Erfindung erlaubt die Herstellung der Verbindungen vom Typ I, die bislang nur in mehrstufigen Synthesen mit zum Teil schlechten Ausbeuten und aufwendigen Reinigungsoperationen zugänglich waren, auf einfachem und wirtschaftlichem Wege in guter Ausbeute, hoher Raum-Zeit-Ausbeute, sowie ausgezeichneter Reinheit. Gerade der Herstellung sehr reiner Produkte kommt in diesem Falle besondere Bedeutung zu, da die nach dem Verfahren der Erfindung hergestellten 2-Acyl-cyclohexan-1,3-dion-Derivate wertvolle Zwischenprodukte für hoch wirksame herbizide Wirkstoffe darstellen (DE-OS 2 822 304, 2 439 104, 3 032 973, 3 047 924, 3 121 355, EP 66 195).

Beispiel 1

Herstellung von 2-Butyryl-5-(p-toluyl)-cyclohexan-1,3-dion

In 1 l Toluol werden 132 g Dimethylmalonat vorgelegt und bei Raumtemperatur mit 180 g 30 %iger Natriummethylat-Lösung versetzt. Es entsteht ein Kristallbrei, zu dem unter kräftigem Rühren 160 g p-Methylbenzalaceton getropft werden. Das Reaktionsgemisch wird innerhalb von 3 Stunden aufgeheizt, wobei gleichzeitig Methanol azeotrop abdestilliert wird, bis die Übergangstemperatur 110 °C erreicht hat. Anschließend werden bei 80 bis 90 °C 106 g Buttersäurechlorid zugegeben und kurz nachgerührt. Nach der Zugabe von 5 g 4-N,N-Dimethylamino-pyridin wird 3 bis 4 Stunden bei 100 °C gerührt und danach das Lösungsmittel verdampft. Der Rückstand wird mit einer Lösung von 120 g Natriumhydroxid in 1,5 l Wasser versetzt und 2 Stunden bei 80 °C gerührt. Anschließend wird bei 60 °C mit 270 ml konz. Salzsäure angesäuert, der Niederschlag nach dem Abkühlen auf Raumtemperatur abgesaugt und mit Wasser neutral gewaschen. Nach dem Trocknen erhält man 239 g (88 %) 2-Butyryl-5-(p-toluyl)-cyclohexan-1,3-dion vom Schmelzpunkt 76 bis 78 °C.

Beispiel 2

Herstellung von 2-Propionyl-5-(pyrid-3'-yl)-cyclo-hexan-1,3-dion

In 1 l Toluol werden 132 g Dimethylmalonat vorgelegt und mit 180 g 30 %iger Natriummethylat-Lösung versetzt. Unter kräftigem Rühren werden anschließend 147 g 1-(Pyrid-3'-yl-but-1 -en-3-on zugetropft). Das Reaktionsgemisch wird innerhalb von 3 Stunden aufgeheizt, wobei gleichzeitig Methanol abdestilliert wird, bis die Übergangstemperatur 110°C erreicht hat. Anschließend werden bei 80°C 92,5 g Propionsäurechlorid zugegeben und 1 h bei 80 °C nachgerührt. Nach Zugabe von 5 g 4-N,N-Dimethylamino-pyridin wird 4 h bei 100°C gerührt. Nach Abkühlen auf Raumtemperatur extrahiert man das Reaktionsgemisch zweimal mit insgesamt 1,2 kg 10%iger Natronlauge und rührt dann den alkalischen Extrakt 3 Stunden bei 60 °C. Nun versetzt man das Reaktionsgemisch mit 365 g 30 prozentiger Salzsäure und rührt 2 Stunden bei 50 °C nach. Nach dem Abkühlen

auf Raumtemperatur wird der ausgefallene Feststoff abgesaugt, mit Wasser gewaschen und getrocknet. Man erhält 189 g (77 %) 2-Propionyl-5-(pyrid-3'-yl)-cyclohexan-1,3-dion vom Schmelzpunkt 80 bis 81 °C.

Beispiel 3

Herstellung von 2-Butyryl-5-cyclohexyl-cyclohexan-1,3-dion

In 1 l Toluol werden 132 g Dimethylmalonat, 180 g 30 %ige Natriummethylat, 152 g 1-Cyclohexyl-but-1-en-3-on und 106,5 g Buttersäurechlorid in analoger Weise wie in Beispiel 1 beschrieben umgesetzt. Zum so erhältlichen Reaktionsgemisch gibt man 5 g 4-N,N-Dimethylaminopyridin und rührt 4 Stunden bei 100°C nach und kühlt dann auf Raumtemperatur. Nun wird in zwei Portionen mit insgesamt 3,5 Mol Natriumhydroxid in 1,5l Wasser extrahiert und die vereinigten alkalischen Auszüge 2 Stunden bei 80 °C gerührt. Anschließend wird bei 50 °C mit ca. 270 ml conz. Salzsäure angesäuert, das anfallende Öl mit Methylenchlorid extrahiert, die Extrakte mit Wasser gewaschen und im Vakuum eingeengt. Man erhält 222 g (84 %) 2-Butyryl-5-cyclohexyl-cyclohexan-1,3-dion als hellbraunes Öl.

Unter entsprechender Abwandlung der Angaben in den vorstehenden Beispielen konnten z. B. die in der nachstehenden Tabelle aufgeführten Verbindungen (I) erhalten werden :

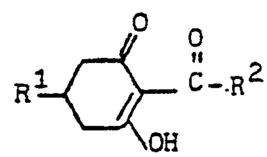

(I)

| $R^1$ | $R^2$ | Schmp. | Ausbeute |
|---|---|---|---|
| $CH_3-CH=CH-$ | $C_3H_7$ | Öl | 72 % |
| 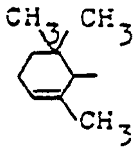 | $C_3H_7$ | Öl | 80 % |
| 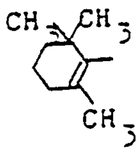 | $C_3H_7$ | Öl | 84 % |
| 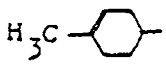 | $C_2H_5$ | Öl | 77 % |
| 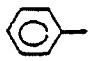 | $C_3H_7$ | 64 - 65°C | 86 % |
| 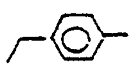 | $C_3H_7$ | 72 - 73°C | 92 % |
| 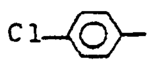 | $C_3H_7$ | | 78 % |
| 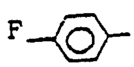 | $C_3H_7$ | 76 - 78°C | 82 % |
| 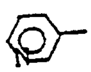 | $C_3H_7$ | 74 - 75°C | 80 % |
| 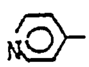 | $C_3H_7$ | 94 - 96°C | 76 % |
| 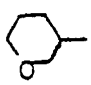 | $C_3H_7$ | 48 - 49°C | 78 % |
| 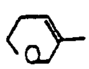 | $C_3H_7$ | 44 - 46°C | 69 % |
| 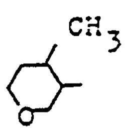 | $C_3H_7$ | 49 - 51°C | 72 % |
| 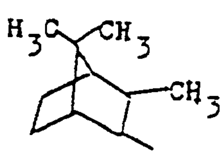 | $C_3H_7$ | 63 - 65°C | 76 % |
| 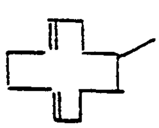 | $C_3H_7$ | 54 - 56°C | 72 % |

(Fortsetzung)

| $R^1$ | $R^2$ | Schmp. | Ausbeute |
|---|---|---|---|
| 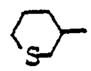 | $C_2H_5$ | 70 - 72°C | 83 % |
| 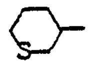 | $C_3H_7$ | 70 - 72°C | 81 % |
| 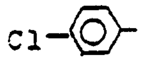 $S-CH_2-CH_2-$ | $C_3H_7$ | öl | 86 % |
| $C_2H_5-S-CH-CH_2-$<br>$CH_3$ | $C_3H_7$ | öl | 89 % |

**Patentansprüche**

1. Verfahren zur Herstellung von Cyclohexandionderivaten der allgemeinen Formel I

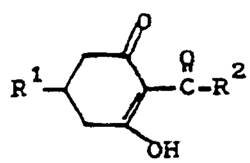

(I)

in der $R^1$ einen der folgenden Substituenten bedeutet: Alkyl mit $C_2$ bis $C_8$, Alkenyl mit $C_2$ bis $C_8$, gegebenenfalls bis zu 4fach olefinisch ungesättigtes Cycloalkyl mit $C_3$ bzw. $C_5$ bis $C_{12}$, Alkylthioalkyl mit $C_2$ bis $C_8$, gegebenenfalls bis zu 3fach olefinisch ungesättigtes Bicycloalkyl mit $C_6$ bis $C_{12}$, gegebenenfalls substituiertes Aryl und Hetaryl oder einen heterocyclischen Rest mit 4 bis 7 Atomen, der bis zu 3 Heteroatome aus der Gruppe O, S und N aufweist und gesättigt oder olefinisch ungesättigt sein kann, durch Umsetzung von α,β-ungesättigten Ketonen der Formel II

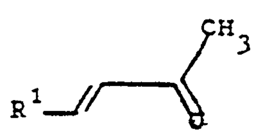

(II)

mit einem Malonsäuredialkylester in Gegenwart einer Base zum Alkoxycarbonylcyclohexenolon bzw. dessen Salz (III)

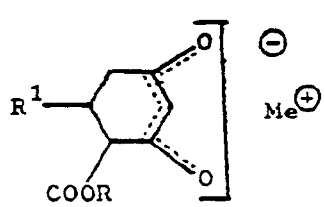

(III)

Acylierung, Verseifung und Decarboxylierung von (III), wobei R den Alkoholrest des Malonsäureesters und $R^1$ die vorstehende Bedeutung hat, dadurch gekennzeichnet, daß man

1. das α,β-ungesättigte Keton (II) mit dem Malonsäuredialkylester in Gegenwart einer Base in einem Lösungsmittel umsetzt, aus dem der freiwerdende Alkohol des Malonsäureesters abdestilliert werden kann,
2. den Alkohol gegebenenfalls als Azeotrop abdestilliert,
3. das Salz des Alkoxycarbonylcyclohexenolons mit einem Carbonsäurehalogenid $R^2COHal$ umsetzt, wobei $R^2$ einen bis zu 6 C-Atomen aufweisenden Alkylrest bedeutet,
4. gegebenenfalls nach Entfernen überschüssigen Acylhalogenids Fertiären Amin als Umlagerungs Katalysator behandelt,
5. verseift und decarboxyliert.

## Claims

1. A process for the preparation of cyclohexanedione derivatives of the general formula I

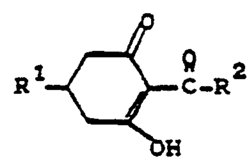

(I)

where $R^1$ is $C_2$-$C_8$-alkyl, $C_2$-$C_8$-alkenyl, $C_3$-$C_{12}$-cycloalkyl or $C_5$-$C_{12}$-cycloalkyl with 0-4 olefinically unsaturated bonds, $C_2$-$C_8$-alkylthioalkyl, $C_6$-$C_{12}$-bicycloalkyl with 0-3 olefinically unsaturated bonds, unsubstituted or substituted aryl or hetaryl, or a heterocyclic radical of 4 to 7 atoms of which not more than 3 may be hetero atoms chosen from O, S and N, the radical being saturated or olefinically unsaturated, by reaction of an α, β-unsaturated ketone of the formula II

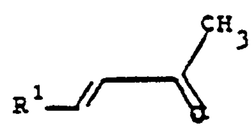

(II)

with a dialkyl malonate in the presence of a base to give the alkoxycarbonylcyclohexenolone or its salt (III)

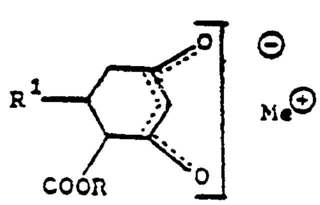

(III)

and acylation, hydrolysis and decarboxylation of (III), R being the alcohol radical of the malonate and $R^1$ having the above meaning, wherein

1. the α,β-unsaturated ketone (II) is reacted, in the presence of a base, with the dialkyl malonate in a solvent from which the alcohol liberated from the malonate can be distilled off,
2. the alcohol is distilled off, where appropriate as an azeotrope,
3. the salt of the alkoxycarbonylcyclohexenolone is reacted with a carboxylic acid halide $R^2$ COHal, where $R^2$ is alkyl of not more than 6 carbon atoms,
4. the product, where appropriate after removal of excess acyl halide, is treated with a tertiary amine as a catalyst for a rearrangement reaction and
5. this product is hydrolyzed and decarboxylated.

## Revendications

1. Procédé de préparation de dérivés de cyclohexandione de formule générale

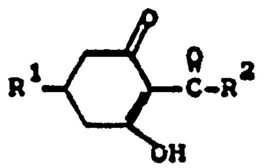

(I)

dans laquelle $R^1$ représente un des substituants suivants : alkyle en $C_2$ à $C_8$, alcényle en $C_2$ à $C_8$, cycloalkyle en $C_3$ ou $C_5$ à $C_{12}$, éventuellement insaturé oléfiniquement jusqu'à 4 fois, alkylthioalkyle en $C_2$ à $C_8$, bicycloalkyle en $C_6$ à $C_{12}$ éventuellement insaturé oléfiniquement jusqu'à 3 fois, aryle et bétaryle éventuellement substitué, ou un reste hétérocyclique à 4 à 7 atomes, qui présente jusqu'à 3 hétéroatomes du groupe O, S et N et qui peut être saturé ou insaturé oléfiniquement, par transformation de cétones à insaturation $\alpha$, $\beta$ de formule II

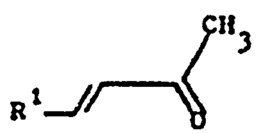

(II)

avec un ester dialkylique d'acide malonique, en présence d'une base, en alcoxycarbonylcyclohexénolone, ou ses sels (III)

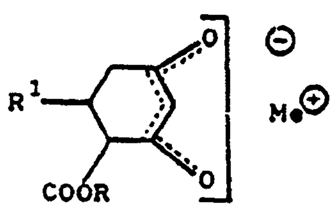

(III)

acylation, saponification et décarboxylation (III), R signifiant le reste alcool de l'ester d'acide malonique et $R^1$ ayant la signification sus-indiquée, caractérisé par le fait que :

1. on fait réagir, dans un solvant, en présence d'une base, la cétone insaturée en $\alpha$ , $\beta$ (II) avec de l'ester alkylique d'acide malonique, dont on peut faire distiller l'alcool libéré de l'ester d'acide malonique,
2. on distille, éventuellement, l'alcool, sous forme d'azéotrope,
3. on fait réagir le sel de l'alcoxycarbonylcyclohexanone avec un halogénure d'acide carboxylique $R^2$-C0Hal, $R^2$ représentant un reste alkyle possédant jusqu'à 6 atomes C,
4. on traite avec un catalyseur d'acylation, éventuellement après élimination d'halogénure d'alkyle en excès, on traite avec une amine tertiaire en tant que catalyseur de transposition,
5. on saponifie et on décarboxyle.